(19) **Europäisches Patentamt / European Patent Office / Office européen des brevets**

(11) **EP 2 261 417 B1**

(12) **EUROPEAN PATENT SPECIFICATION**

(45) Date of publication and mention of the grant of the patent:
**01.08.2018 Bulletin 2018/31**

(21) Application number: **09727531.7**

(22) Date of filing: **25.03.2009**

(51) Int Cl.:
*A61L 9/014* (2006.01)  *A61L 9/16* (2006.01)
*D06M 11/45* (2006.01)  *D06M 11/46* (2006.01)
*D06M 11/79* (2006.01)  *D06M 13/00* (2006.01)
*D06M 13/422* (2006.01)  *D06M 23/08* (2006.01)

(86) International application number:
**PCT/JP2009/055875**

(87) International publication number:
**WO 2009/122975 (08.10.2009 Gazette 2009/41)**

(54) **DEODORANT FIBROUS STRUCTURE AND AIR FILTER**

DESODORIERENDE FASERSTRUKTUR UND LUFTFILTER

STRUCTURE FIBREUSE DÉSODORISANTE ET FILTRE À AIR

(84) Designated Contracting States:
**AT BE BG CH CY CZ DE DK EE ES FI FR GB GR HR HU IE IS IT LI LT LU LV MC MK MT NL NO PL PT RO SE SI SK TR**

(30) Priority: **31.03.2008 JP 2008089932**
**01.07.2008 JP 2008172124**

(43) Date of publication of application:
**15.12.2010 Bulletin 2010/50**

(73) Proprietor: **Toray Industries, Inc.**
**Tokyo 103-8666 (JP)**

(72) Inventors:
• **ASADA, Yasuhiro**
**Otsu-shi**
**Shiga 520-2141 (JP)**

• **ANDO, Keiichi**
**Otsu-shi**
**Shiga 520-2141 (JP)**
• **MIYOSHI, Kengo**
**Otsu-shi**
**Shiga 520-8558 (JP)**

(74) Representative: **Webster, Jeremy Mark et al**
**Mewburn Ellis LLP**
**City Tower**
**40 Basinghall Street**
**London EC2V 5DE (GB)**

(56) References cited:
WO-A1-01/62309    WO-A1-2007/066438
WO-A1-2007/074816    JP-A- 10 292 258
JP-A- 10 292 263    JP-A- 11 172 574
JP-A- 2008 148 804    JP-A- 2009 028 207

## Description

### TECHNICAL FIELD

The present invention relates to a fiber structure for deodorization and an air filter.

### BACKGROUND ART

Pollution substances in the air have diverse kinds. Among them, particularly aldehydes such as acetaldehyde etc. have become a great problem. Acetaldehyde is a representative offensive odor component contained in a tobacco smoke and an exhaust gas of automobiles; having a low threshold, an odor of acetaldehyde is easily sensed even at a low concentration and, additionally, adverse influence on a human body has been pointed out. For removing an offensive odor component in the air, activated carbon having a great surface area and pore volume has previously been used generally. However, equilibrium adsorption amount of acetaldehyde onto activated carbon is remarkably small as compared with other offensive odor components, and the activated carbon has no practical performance.

Then, as a means for improving performance of removing adsorption of acetaldehyde with activated carbon, for example, a method adhering amines to activated carbon to improve performance thereof has been proposed (see Patent Literature 1). However, an air filter using that technique had a problem that an odor caused by substances other than acetaldehyde is generated by change in temperature, humidity etc. (secondary odor emission). That is, since activated carbon is based on the physisorbing ability, it adsorbs and concentrates also substances other than acetaldehyde to be removed. However, since these odor components are not trapped by a chemical bond, odor components which have been concentrated are released at once by environmental factors such as change in temperature or humidity, etc. As a result, an odor component which did not become a problem at the original presence concentration is recognized as an offensive odor.

In addition, as a means for adsorption and removal of acetaldehyde not using activated carbon, a fiber sheet supporting porous silica containing mainly meso pores and acid hydrazides on a fiber surface has been disclosed (see Patent Literature 2). Since this fiber sheet has the excellent ability to remove acetaldehyde and, at the same time, hardly causes physisorption of an organic gas as in activated carbon, it considerably decreases a secondary odor emission risk. However, conventional porous silica has strong hygroscopicity. For this reason, water-soluble odor components are adsorbed and released with moisture which is adsorbed and desorbed by porous silica, and secondary odor emission has not been completely suppressed even by this technique.

> Patent Literature 1: JP-A No. 5-317703 gazette
> Patent Literature 2: JP-A No. 2007-167632 gazette WO2007/074816 A1 describes an air purifier for removing aldehyde from air. WO2007/066438 A1 describes an odor eliminating cloth.

### DISCLOSURE OF THE INVENTION

Problems to be solved by the invention

An object of the present invention is to provide a fiber structure for deodorization, which effectively removes aldehydes such as acetaldehyde etc., and has extremely small secondary odor emission risk.

Means to solve the problems

The present invention for solving the aforementioned problems comprises a fiber structure and air filter according to the claims. Also described are any of the following constructions.

> (1) A fiber structure for deodorization, wherein inorganic particles and water-soluble amine compound are supported in a fiber structure, an equilibrated water content under the environment adjusted at a temperature of 25°C and a relative humidity of 75% is 15% by mass or less, and a pH when the fiber structure is immersed in water to 3% by weight relative to water is 3.5 to 7.
> (2) The fiber structure for deodorization according to (1), wherein an equilibrated water content of the inorganic particles under the environment adjusted at a temperature of 25°C and a relative humidity of 75% is 10% by mass or less.
> (3) The fiber structure for deodorization according to (1) or (2), wherein the inorganic particles are hydrophobic porous particles.
> (4) The fiber structure for deodorization according to (3), wherein the hydrophobic porous particles have an average pore diameter of 2 to 50 nm.

(5) The fiber structure for deodorization according to any one of (1) to (4), wherein the inorganic particles and the water-soluble amine compound form pores on a surface of a fiber, a volume occupied by pores having a pore diameter of 20 nm or less is 40% or less of a total pore volume, and a specific surface area of the fiber structure is 1 to 30 m$^2$/g.

(6) The fiber structure for deodorization according to any one of (1) to (5), wherein the inorganic particles have a number average particle diameter of 1 $\mu$m or less and a specific surface area of 15 to 250 m$^2$/g by BET method.

(7) The fiber structure for deodorization according to any one of (1) to (6), wherein the inorganic particles contain at least one kind selected from the group consisting of silica, alumina and titania.

(8) The fiber construction for deodorization according to any one of (1) to (7), wherein the water-soluble amine compound contains an acid hydrazide compound.

(9) The fiber structure for deodorization according to any one of (1) to (8), wherein a water-insoluble pH control agent is contained.

(10) The fiber structure for deodorization according to (9), wherein the water-insoluble pH control agent is a flame retardant.

(11) The fiber structure for deodorization according to any one of (1) to (10), wherein the inorganic particles and the water-soluble amine compound are supported on a fiber surface with a binder.

(12) The fiber structure for deodorization according to any one of (1) to (10), wherein the inorganic particles have a number average particle diameter of 50 to 2000 $\mu$m and is held between fibers in the state where the water-soluble amine compound is adhered to the inorganic particles.

(13) An air filter constructed with the fiber structure for deodorization according to any one of (1) to (12).

Effects of the invention

The fiber structure for deodorization of the present invention is excellent in a reaction velocity with aldehydes and in an adsorption volume. Furthermore, inorganic particles supported by the fiber structure for deodorization, unlike activated carbon, show extremely small physisorption, and are suppressed also in hygroscopicity as compared with conventional inorganic porous body. For this reason, regarding gas components other than aldehydes, an adsorption concentration is suppressed whether hydrophilic or hydrophobic, and so even when temperature and humidity of the usage environment fluctuate wildly, there is a low possibility that an odor component is re-released (secondarily odor emission). Therefore, the fiber structure can exert an excellent performance particularly in air filter utility.

BRIEF DESCRIPTION OF THE DRAWINGS

Fig. 1 is a graph showing change with time in an acetaldehyde removal efficiency of the fiber structure for deodorization in Examples.

Fig. 2 is a graph showing change with time in an acetaldehyde removal efficiency of the fiber structure for deodorization in Comparative Examples.

BEST MODE FOR CARRYING OUT THE INVENTION

The fiber structure for deodorization of the present invention is importantly such that inorganic particles and water-soluble amine compound are supported, and an equilibrated water content under the environment adjusted at a temperature of 25°C and a relative humidity of 75% is 15% by mass or less.

The fiber sheet of the prior art using a porous body containing not activated carbon, but mainly containing a meso pore, for example, porous silica considerably reduces secondary odor emission as compared with the sheet using activated carbon, but cannot completely suppress secondary odor emission. The present inventors pinned down that this is caused by the property of porous silica. That is, since porous silica containing mainly a meso pore has a overwhelmingly small micro pore ratio as compared with activated carbon, it hardly produces physisorption of an organic gas etc., but since chemical property of a pore surface is hydrophilic, it has strong hygroscopicity. The present inventors pinned down that a water-soluble odor component is once dissolved in water adsorbed by porous silica water is removed from porous silica again, and therewith, secondary odor emission is caused.

Then, in the present invention, in order to suppress adsorption of water by a deodorizer, which causes secondary odor emission, the following two methods were adopted to make inorganic particles and water-soluble amine compound supported in a fiber structure for deodorization and, at the same time, to make an equilibrated water content of the fiber structure for deodorization 15% by mass or less. Specifically, a first method is a method using hydrophobic porous particles in which a pore surface of porous particles is modified to be hydrophobic (hereinafter, referred to as first method). And, a second method is a method of forming a pore structure having a pore diameter controlled in a specified range, by coagulating inorganic particles having a number average particle diameter of 1 $\mu$m or less (hereinafter, referred to as second method). By these methods, it becomes possible to suppress secondary odor emission of a water-soluble odor component while an effective area as a reaction field of an added chemical (chemisorption of aldehydes by amine compound) is maintained.

First, inorganic particles in the first method will be explained.

When the first method is adopted, water-soluble amine compound described later can be adhered at a sufficient amount, due to the presence of hydrophobic porous particles. As a result, a surface area in which a treating air and water-soluble amine compound can be contacted, can be enhanced, and an efficiency of removal of aldehydes can be increased.

The average pore diameter of the hydrophobic porous particles is 2 to 50 nm. By making an average pore diameter 2 nm or more, more preferably 5 nm or more, adhesion of water-soluble amine compound into the interior of pores becomes possible, and diffusion of the gas into pores is easy; thus, contact between an adhered chemical and a subject gas is performed better, and a reaction can be proceeded effectively. In addition, by making an average pore diameter 50 nm or less, more preferably 30 nm or less, a necessary surface area as a reaction field of an adhered chemical can be maintained.

In addition, it is preferable that the hydrophobic porous particles have a specific surface area by a BET method of 50 to 600 $m^2$/g. By making a specific surface area 50 $m^2$/g or more, more preferably 100 $m^2$/g or more, an effective area as a reaction field of a chemical to be added is obtained, and an effective reaction velocity with a gas component to be removed is obtained. In addition, by making a specific surface area 600 $m^2$/g or less, more preferably 500 $m^2$/g or less, inconvenience of handling property due to decrease in a mechanical strength of porous particles can be prevented. Here, the specific surface area by a BET method refers to a value measured according to a BET multiple point method defined in JIS R1626-1996 (adsorbate: $N_2$, constant volume method).

Furthermore, such the porous particles can prevent adsorption and desorption of water which causes secondary odor emission, by making a pore surface of such the porous particles hydrophobic. As an extent of hydrophobicity of hydrophobic porous particles, an equilibrated water content under the environment adjusted at temperature of 25°C and a relative humidity of 75% is preferably 10% by mass or less, more preferably 8% by mass or less. On the other hand, since extreme hydrophobicity exhibiting water repellency makes difficult for a water-soluble amine-based drug described later to adhere to the interior of pores of inorganic particles; from such the viewpoint, it is preferable that the equilibrated water-content is 2% by mass or more.

As the hydrophobic porous particles, hydrophobic porous silica is preferable. Examples of the inorganic porous body include porous silica, zeolite, activated alumina, aluminum silicate, alumina gel, activated clay, zirconium phosphate, ammonium tripolyphosphate etc., and usually many of these inorganic porous bodies are hydrophilic. However, a hydrophobic degree of a surface of porous silica can be adjusted by controlling the number of silanoles on a pore surface. Since the silanole is a porous group which easily makes a hydrogen bond with other molecule, it selectively adsorbs a polar molecule, a representative of which is a water molecule, and influences on a hydrophobic degree. As a method of decreasing silanoles of porous silica, there are a method of reacting the silanole with a hydrophobic compound such as a silane coupling agent etc., and a method of condensing the silanole by heat treatment at about 400°C. However, in a method of reacting the silanole with another compound, extreme hydrophobicity in which inorganic particles after treatment exhibit water repellency is easily generated, and it becomes difficult to make a water-soluble amine-based drug described later adhere into the interior of a pore of inorganic particles. For this reason, a method of decreasing silanoles by heat treatment is more preferable.

A number average particle diameter of the hydrophobic porous particle is preferably 0.01 to100 $\mu$m, more preferably 0.1 to 50 $\mu$m. By making a number average particle diameter 100 $\mu$m or less, further preferably 50 $\mu$m or less, an efficiency of contact with a treating air can be increased and, at the same time, the particles can be uniformly supported on a fiber surface. On the other hand, by making a number average particle diameter 0.01 $\mu$m or more, more preferably 0.1 $\mu$m or more, particles are prevented from being embedded in a binder resin etc. used for supporting it on a fiber surface. As a method of measuring a number average particle diameter, for example, a method of measuring a particle diameter (on number basis) by observation using SEM (scanning electron microscope) can be adopted.

A supporting amount of hydrophobic porous particle in the fiber structure for deodorization of the present invention is preferably 10 to 100% by mass relative to a fiber structure of a substrate. By making the amount 10% by mass or more, more preferably 20% by mass or more, the adsorption performance can be improved by obtaining an effective surface area as a reaction field of a chemical. In addition, by making the amount 100% by mass or less, more preferably 50% by mass or less, inhibition of air permeability property as an air filter can be prevented.

Then, inorganic particles in a second method will be explained.

In the second method of suppressing adsorption of water by the fiber structure for deodorization, a pore structure having a pore diameter controlled in a specified range is formed in the fiber structure, by coagulating inorganic particles on a surface of a fiber. Further, by controlling the pore structure so that a volume occupied by pores having a pore diameter of 20 nm or less becomes 40% or less of a total pore volume, adsorption of water which causes secondary odor emission can be suppressed.

In addition, in the pore structure obtained by coagulation of inorganic particles, by making a specific surface area $1.00 m^2/g$ or more, an effective area as a reaction field of a chemical to be added (chemisorption of aldehydes by amine compound) is obtained, and an effective reaction velocity with a gas component to be removed is obtained. By making a specific surface area $30.00 m^2/g$ or less, adsorption and desorption of water and a water-soluble odor component which causes secondary odor emission can be suppressed.

In the fiber structure for deodorization obtained by the second method, inorganic particles are coagulated on a fiber surface of a fiber structure which is to be a substrate, to form pores where amine compound is supported. Generally, pores are classified into a macro pore having a pore diameter of more than 50 nm, a meso pore having a pore diameter of 2 to 50 nm, and a micro pore having a pore diameter of less than 2 nm, according to the specification of IUPAC (International Union of Pure and Applied Chemistry) . As a pore diameter approaches a micro pore side, hygroscopic property of water is strengthened, and water becomes easy to be adsorbed and accumulated. The second method does not rely on a porous structure of inorganic particles by themselves, but utilizes a pore structure formed by coagulation of inorganic particles. For this reason, a ratio of a pore volume occupied by a pore near a micro pore in a total pore volume becomes small as compared with a ratio of a pore volume occupied by a pore near a micro pore when particles themselves are a porous body and, as a result, it is thought that adsorption of water can be suppressed. Specifically, by suppressing a volume occupied by a pore having a pore diameter of 20 nm or less to 40% or less of a total pore volume, it becomes possible to suppress adsorption of water which causes secondary odor emission. Here, a pore volume and a pore diameter distribution can be obtained by a BET method. In addition, when inorganic particles are a porous structure, a pore referred herein also includes a pore of the inorganic particles themselves.

Inorganic particles in the second method are ultrafine particles having a number average particle diameter of 1 $\mu$m or less. By adopting such the ultrafine particles, inorganic particles can be supported so as to cover a fiber surface of the fiber structure, and a sufficient amount of a water-soluble amine compound described later can be adhered on a surface of the inorganic particles. As a result, a surface area in which contact between a treating air and water-soluble amine compound is possible can be increased, and an efficiency of removing aldehydes can be enhanced. On the other hand, in order to prevent inorganic particles being embedded in a binder resin used in supporting the particles on a fiber surface, it is preferable that a number average particle diameter of the ultrafine particle is 0.005 $\mu$m or more, more preferably 0.01 to 0.5 $\mu$m. Here, as a method of measuring a number average particle diameter, for example, a particle diameter measuring method (on a number basis) by observation using SEM (scanning electron microscope) can be adopted.

In addition, it is preferable that the ultrafine particles are inorganic particles which are relatively easily blended with water, from a viewpoint of adhering property with a drug described later.

Examples of a material of the ultrafine particles include silica, alumina, and titania; among them, the material can be selected depending on the purpose. Inter alia, silica and alumina are preferable. Alternatively, these ultrafine particles can be also used by mixing them.

Examples of a shape of the ultrafine particles include globular, elliptical, needle-like, bar-like, scale-like, plate-like, and ground-like.

In addition, the ultrafine particles adopted in the present invention have a specific surface area by a BET method of preferably 15.00 to 250.0 $m^2/g$, more preferably 30.00 to 200.0 $m^2/g$. By making a specific surface area 15.00 $m^2/g$ or more, an effective area as a reaction field of a chemical to be added (chemical adsorption of aldehydes by water-soluble amine compound) is obtained, and an effective reaction velocity with a gas component to be removed is obtained. In addition, by making a specific surface area 250.0 $m^2/g$ or less, adsorption and desorption of water and a hydrophilic gas which causes secondary odor emission can be suppressed. Here, the specific surface area by a BET method refers to a value measured according to a BET multiple point method as defined in JIS R1626-1996 (adsorbate: $N_2$, constant volume method).

An equilibrated water content under the environment of a temperature of 25°C and a relative humidity of 75% of the ultrafine particles is preferably 10% by mass or less, more preferably 8% by mass or less. By making an equilibrated water content 10% by mass or less, it becomes possible to suppress adsorption of water and a hydrophilic gas onto the ultrafine particles, and it becomes possible to suppress secondary odor emission.

The surface chemical property of the ultrafine particles may be hydrophilic, or may be hydrophobilicization-treated by reacting a hydroxyl group of the ultrafine particle with a silane coupling agent. Specific examples of the silane coupling agent include epoxysilane and aminosilane.

A supporting amount of the ultrafine particles is preferably 10 to 100% by mass, more preferably 20 to 50% by mass relative to a mass of the fiber structure which is a substrate. By making the amount 10% by mass or more, adsorption performance can be improved by obtaining an effective surface area as a reaction field of a chemical. By making the amount 100% by mass or less, inhibition of air permeability property as an air filter can be prevented.

Then, as a fiber constituting the fiber structure, the same fiber can be used in the first method and the second method. Specifically, natural fibers, synthetic fibers, or inorganic fibers such as glass fibers, metal fibers etc. can be used and; inter alia, synthetic fibers of a thermoplastic resin which can be melt-spun are preferably used.

Examples of the thermoplastic resin forming such the synthetic fibers include polyester, polyamide, polyolefin, acryl, vinylon, polystyrene, polyvinyl chloride, polyvinylidene chloride, polylactic acid etc., and can be selected and used depending on the utility etc. Alternatively, a plurality of kinds may be used by combination.

As such the synthetic fibers, for example, a fiber having a modified cross-sectional shape, or a fiber having many pores or slits on a fiber surface is preferably used. By forming into such the shape, a surface area of a fiber can be increased, and property of carrying inorganic particles such as the hydrophobic porous particles and the ultrafine particles, and the water-soluble amine compound can be improved. Here, the modified cross-sectional shape referred herein refers to a cross-sectional shape other than a circle, and examples include a flat type, an approximate polygon, a wedge shape etc. A fiber of such the modified cross-sectional shape can be obtained by spinning using a spinneret having a modified pore. In addition, the fiber having many pores or slits on a fiber surface can be obtained by alloying two or more kinds of polymers having different solubilities in a solvent, spinning the polymer alloy, and dissolving and removing a polymer having high solubility with a solvent.

A fiber diameter of a fiber constituting the fiber structure may be selected depending on goal air permeability and dust collecting performance in utility in which the fiber is used as an air filter, and is preferably 1 to 2000 $\mu$m. By making a fiber diameter 1 $\mu$m or more, more preferably 5 $\mu$m or more, clogging of inorganic particles on a fiber structure surface can be prevented, and reduction in air permeability can be prevented. In addition, by making a fiber diameter 2000 $\mu$m or less, more preferably 100 $\mu$m or less, reduction in the ability to support the inorganic particles and reduction in an efficiency of contact with a treating air due to decrease in a fiber surface area can be prevented.

As a form of the fiber structure, a cotton-like fabric, a knitted or woven fabric, a non-woven fabric, a paper and other three-dimensional net (e.g. a porous polyurethane foam, or a structure of monofilament forming three-dimensional loops irregularly and piled) can be used. In brief, a fiber structure which can achieve a great surface area while air permeability suitable for use as an air filter is maintained, may be used.

A weight of the fiber structure is preferably 10 to 500 g/m$^2$, more preferably 30 to 200 g/m$^2$. By making a weight 10 g/m$^2$ or more, more preferably 30 g/m$^2$ or more, a sufficient strength to withstand a process of supporting inorganic particles is obtained, and a necessary rigidity for maintaining a filter structure when the air is permeated, is obtained. In addition, by making a weight 500 g/m$^2$ or less, more preferably 200 g/m$^2$ or less, hydrophobic porous particles can be uniformly carried up to the interior of the fiber structure, and handling property upon secondary processing into a pleat or a honeycomb is excellent.

It is important that the fiber structure for deodorization of the present invention has water-soluble amine compound on a surface or in the interior of pores of the inorganic particles, or on a fiber surface. Due to the presence of the water-soluble amine compound, aldehydes such as formaldehyde and acetaldehyde can be selectively adsorbed. Herein, "water-soluble" refers to dissolution of 1% by mass (10 g/L) or more in neutral water at 25°C and, in order to obtain a sufficient supporting amount, more preferable solubility is 5% by mass (50 g/L) or more. By making amine compound water-soluble, it becomes possible that the amine compound is sufficiently supported on a surface or in the interior of pores of inorganic particles, or on a fiber surface. And, by the presence of the water-soluble amine compound on a surface or in the interior of pores of inorganic particles, the chemical adsorbing ability for aldehydes such as formaldehyde and acetaldehyde is dramatically improved.

A chemical adsorption mechanism for aldehydes of the water-soluble amine compound is thought as follows. That is, a lone electron pair possessed by a nitrogen atom of an amino group of the water-soluble amine compound nucleophilically attacks a carbonyl carbon atom of aldehydes, reacts therewith, and fixes this as a derivative such as imine or enamine.

Examples of the water-soluble amine compound include acid hydrazide, hydrazines, aliphatic amines, aromatic amines, ureas and amino acid; among them, acid hydrazide is preferable form a viewpoint of the chemical adsorbing ability for aldehydes . Acid hydrazide is a compound having an acid hydrazide group represented by -CO-NHNH$_2$ which is derivatized from carboxylic acid and hydrazine, and a nitrogen atom further having a lone electron pair is bound to an $\alpha$-position of a nitrogen atom at an end of hydrazide; thereby, nucleophilic reactivity is remarkably improved. Among aldehydes, since acetaldehyde has an electron donating alkyl group at an $\alpha$-position of carbonyl carbon, electrophilicity of carbonyl carbon is low, and it is chemically adsorbed with difficulty. However, since acid hydrazides have high nucleophilic reactivity as described above, they manifest the better chemical adsorbing performance also for acetaldehyde.

Examples of acid hydrazide include carbonic dihydrazide, adipic acid dihydrazide, succinic acid dihydrazide, glutamic acid dihydrizide etc.

A supporting amount of the water-soluble amine compound is preferably 2% by mass or more relative to a mass of inorganic particles, except for the case where deodorizer particles are pre-formed as described below. By making the amount 2% mass or more, more preferably 4% by mass or more, further 10% by mass or more, effectiveness of improvement in an efficiency of removing aldehydes, and an adsorption volume can be obtained. As a supporting amount of the water-soluble amine compound is increased, a removal rate and an adsorption volume are also improved, but are saturated at some extend. Excessive addition of the water-soluble amine compound causes disintegration of the crystallized water-soluble amine compound decreasing a void rate of the fiber structure, reducing air permeability property, and powder dropping at the same time,. For this reason, the amount is preferably 200% by mass or less, more preferably 150% by mass or less, further preferably 100% by mass or less.

In addition, for the purpose of improving the aldehyde removing performance, it is also preferable to add other water-soluble amine compound in addition to the acid hydrazide compound. When the acid hydrazide compound and guanidine sulfamate are used together, the particularly excellent effect is exerted.

A supporting amount of guanidine sulfamate to be used together is preferably 0.5 to 2.5% by mass relative to the fiber structure. By making the amount 0.5% by mass or more, effectiveness of improvement in an efficiency of removing aldehydes and an absorption volume can be obtained. Since guanidine sulfamate shows hygroscopicity, by making the amount 2.5% by mass or less, a moisture absorbing amount of the fiber structure can be suppressed, and a water amount which causes secondary odor emission can be suppressed.

When acid hydrazide compound and guanidine sulfamate are used together, a mass ratio of guanidine sulfamate relative to acid hydrazide compound is preferably 0.05 to 0.20.

In addition, it is preferable that the fiber structure for deodorization of the present invention contains a binder resin. The binder resin makes the fiber structure support inorganic particles firmly. In addition, use of the binder resin is preferable also in respect of shape retainability, a strength, dimensional stability etc. of the fiber structure for deodorization.

Examples of the binder resin include acrylic, acrylstyrene, acrylsilicone, polyurethane, polyester, polyamide, polyolefin, ethylene-vinyl acetate copolymer resins etc. As an aspect of the binder resin, any of an emulsion, a dispersion, a powder etc. can be used; in order to support fine inorganic particles on a fiber surface firmly, an emulsion is preferable. Inter alia, an acrylic emulsion resin is preferably used since the aldehyde removing performance is excellent.

An additive amount of the binder resin is preferably 5 to 200% by mass relative to inorganic particles. By making the amount 5% by mass or more, more preferably 10% by mass or more, fixability of inorganic particles on the fiber structure can be improved. In addition, by making the amount 200% by mass or less, more preferably 100% by mass or less, inorganic particles can be prevented from being embedded in or covered with the binder resin to inhibit contact with a gas.

In addition, the fiber structure for deodorization of the present invention is importantly such that a pH when immersed in water to 3% by mass is 3.5 to 7. Thereby, performance of removing aldehydes is improved. By making a pH 7 or lower, preferably 6.5 or lower, an intermediate produced from a reaction by nucleophilic attack against a carbonyl carbon atom of aldehydes with a lone electron pair of amine compound is easily protonated and dehydrated in an acidic reaction field, and a reaction of fixing into a derivative sufficiently proceeds. In addition, by making a pH 3.5 or higher, preferably 4 or higher, the activity of a lone electron pair of amine compound which nucleophilically attacks a carbonyl carbon atom of aldehydes can be sufficiently maintained.

A pH of the fiber structure for deodorization can be adjusted, for example, by adding a pH control agent, or selecting a binder resin having a suitable pH as the binder resin.

It is preferable to adopt, as the pH control agent, a pH control agent which does not generate an odor by itself, or a pH control agent which shows low hygroscopicity. When a pH control agent is added by mixing with an aqueous dispersion, a pH control agent having small water-solubility (which is water-insoluble) is preferable because stability of a dispersion can be retained. Here, a water-insoluble pH control agent refers to a substance which is dissolved in neutral water at 25°C by less than 1% by mass (10 g/L).

Specific examples of the water-insoluble pH control agent include the following. For example, when adipic acid dihydrazide is used as the water-soluble amine compound, and the fiber structure is impregnation-processed with such the water-soluble amine compound and inorganic particles with the binder resin as an aqueous dispersion, adipic acid can be preferably adopted. Since adipic acid retains balance of the aqueous dispersion stable and is not accompanied with generation of an odor and manifestation of hygroscopicity, it is preferable. In addition, phosphoric acid melamine can be also preferably adopted because there is neither generation of an odor nor manifestation of hygroscopicity. Furthermore, phosphoric acid melamine also functions as a flame retardant and can improve the flame retardant property of the fiber structure for deodorization.

The fiber structure for deodorization of the present invention as described above can be obtained, for example, by any of the following methods. That is (i) a method of preparing an aqueous dispersion of inorganic particles and water-soluble amine compound together with a binder, and coating a fiber with them simultaneously, (ii) a method of making inorganic particles supported on a fiber surface in advance, and coating a fiber with an aqueous solution of water-soluble amine compound, (iii) a method of making deodorizer particles in which water-soluble amine compound is adhered to inorganic particles, and making the deodorizer supported on a fiber surface etc.

Specifically, in (i), an aqueous dispersion containing the inorganic particles, the water-soluble amine compound, further preferably, the binder-resin, and if necessary, a pH of which is adjusted at 3.5 to 7 with a pH control agent, is coated on a fiber structure to make the structure impregnated therewith, and the structure is dried; thereby, the structure is obtained. In addition, as another method, for example, the aqueous dispersion may be coated, or sprayed on a fiber structure, followed by drying. According to these methods, when the inorganic particles and the water-soluble amine compound and, further, the pH control agent are added, a fiber structure for deodorization in which the pH control agent is supported on a fiber surface with the binder is obtained.

In (ii), inorganic particles are fixed on the fiber surface of a fiber structure in advance, and thereafter, an aqueous solution obtained by mixing water-soluble amine compound and a pH control agent may be adhered by dipping treatment or spray treatment. As a method of imparting the inorganic particles to a fiber surface in this case, a method of adhesion-supporting with a binder resin may be used, or the inorganic particles may be arranged on a fiber surface at a yarn-making stage. For example, in core/sheath conjugate spinning of a synthetic fiber, by blending a large amount of porous inorganic particles in a sheath component, the inorganic particles can be unevenly distributed in vicinity of a surface of a core/sheathe conjugate. In addition, it is also preferable that, in this method, a suitable amount of a resin component on a surface is removed by chemical or physical treatment, in order to expose the inorganic particles on a surface.

In (iii), deodorizer particles carrying the water-soluble amine compound and the pH control agent in inorganic particles in advance are obtained, and this may be attached to the fiber structure with a binder resin etc. The deodorizer particles can be obtained by impregnating the inorganic particles with an aqueous solution in which the water-soluble amine compound and the pH control agent are dissolved or dispersed and, thereafter, drying this. According to such the method, the fiber structure for deodorization in which the deodorizer particles with the water-soluble amine compound and the pH control agent added to the inorganic particles are held and incorporated between fibers is obtained.

Upon production of deodorizer particles in advance, as the inorganic particles, the aforementioned inorganic particles can be fundamentally employed. It is a preferable that the inorganic particles are molded into a particle size which is easily handled as a deodorizer and, thereafter, the water-soluble amine compound drug and the pH control agent are attached. It is preferable that the water-soluble amine compound is added at a relatively low temperature after particle molding, in order to avoid deterioration of performance due to thermal degradation etc.

Examples of a method of molding the deodorizer particles include a method of granulating a mixture of inorganic particles, a binder and a heat-fusing resin, and heating and molding this, or impregnating a particulate porous substrate with a slurry containing inorganic particles, a binder and a heat-fusing resin, and heat-integrating this.

A number average particle diameter suitable as the deodorizer particles is preferably 50 to 2000 $\mu$m, more preferably 80 to 1000 $\mu$m. By making the particle diameter 2000 $\mu$m or less, it becomes possible to adhere a chemical even into the interior of the deodorizer particles; by making the particle diameter 50 $\mu$m or more, flying is suppressed, handling as a deodorizer becomes easy, and the fiber structure for deodorization of the present invention can be obtained by dispersing the deodorizer particles between fibers, or holding the deodorizer particles between a plurality of substrate fibers. Further, by making the deodorizer particles 1000 $\mu$m or less, post processing such as bending processing etc. of the fiber structure for deodorization becomes possible; by making the deodorizer particles 100 $\mu$m or more, a deodorizer can be prevented from dropping from a void of the fiber structure for deodorization.

As the water-soluble amine compound to be adhered to the deodorizer particles, fundamentally water-soluble amine compound having the characteristic of the aforementioned water-soluble amine-based drug may be used, and an adhesion amount of the water-soluble amine compound in such the deodorizer is preferably 1 to 50% by mass, more preferably 2 to 40 % by mass relative to inorganic particles constituting the deodorizer. By making the amount 1 % by mass or more, an efficiency of the effect of removing aldehydes and improvement in an adsorption volume can be obtained; by making the amount 50% by mass or less, dropping of a crystallized water-soluble amine-based drug from the deodorizer, and inhibition of diffusion of a gas into the interior of the deodorizer can be avoided.

In addition, as the pH control agent in the deodorizer particles, fundamentally, a pH control agent having characteristics of the aforementioned pH control agent may be used.

It is preferable that the deodorizer particles are dispersed between fibers, or held between a plurality of fiber sheets which are to be a substrate, in forming the fiber structure for deodorization. In the case of the former, for example, a so-called air-laid method sucking a mixed dispersion of the heat-fusing fiber and the deodorizer on a trapping net to web it and, further, fusion-integrating the web in a heating furnace is exemplified. In the case of the latter, for example, the deodorizer particles together with a fibrous or powdery heat adhering body is sprayed at an amount on a fiber sheet, a cover sheet is laminated, and this is heat-pressed, thereby, they can be integrated. In any case, by further using a binder resin together, the deodorizer particles can be supported firmly.

The fiber structure for deodorization of the present invention is suitably used in an air filter. Thereupon, it is also preferable that a fiber structure having a different fiber construction is further laminated on the fiber structure for deodorization of the present invention. For example, in use as a cross flow-type air filter, when a bulky burred non-woven fabric sheet is laminated on an upstream side, a dust retaining amount is increased, and a longer life becomes possible. In addition, when a non-woven sheet consisting of an ultrafine fiber is laminated on a downstream side, a higher trapping rate becomes possible.

Furthermore, it is more preferable that this non-woven fabric sheet consisting of the ultrafine fiber is electret-treated. By being electret-treated, a submicron size or nano size fine dust which is usually removed with difficulty can be trapped by a static electricity force.

As a material constituting such the electret non-woven fabric, materials having a high electric resistivity such as polyolefin resins such as polypropylene, polyethylene, polystyrene, polybutylene terephthalate, polytetrafluoroethylene etc., aromatic polyester resins such as polyethylene terephthalate etc., polycarbonate resins etc. are preferable.

As a shape of the air filter, the air filter may be used as it is in a plane manner, but it is preferable to adopt a pleat-type or a honeycomb-type. A pleat-type, in use as a cross flow-type filter, and a honeycomb-type, in use as a parallel flow-type filter, can increase a contact area of a treating air to improve a trapping efficiency and, at the same time, can achieve a lower pressure drop simultaneously.

In addition, it is preferable that the air filter of the present invention is used by housing in a frame, in respect of an air treating efficiency and handling property.

Examples

[Measurement method]

(1) BET specific surface area and ratio of pore volume of pore diameter of 20 nm or less relative to total pore volume

A specific surface area of the fiber structure for deodorization and the inorganic particles was measured according to a BET multiple point method defined in JIS R 1626-1996 using NOVA 2200e manufactured by YUASA-IONICS. As a sample, about 500 mg of the fiber structure for deodorization, and about 100 mg of the inorganic particles were collected; vacuum degassing was performed at 100°C for 10 hours, and a specific surface area was measured using $N_2$ as an adsorbate by a constant volume method. In addition, a specific surface area was measured down to the third decimal place, and the third decimal place was rounded off to the second decimal place.

In addition, an adsorption amount of a nitrogen gas at a boiling point of liquid nitrogen (-195.8°C) was measured at twenty points while a pressure was gradually raised in a range of a relative pressure of 0.05 to 0.995; a nitrogen adsorption isotherm was produced, a total pore volume of the fiber structure for deodorization and a volume of each pore were measured by a BET method, respectively, and a ratio (%) of a pore volume of a pore diameter of 20 nm or less relative to a total pore volume was obtained.

(2) Average pore diameter

Assuming that a shape of each pore of a porous body was cylindrical, an average pore diameter (D) was calculated from a pore volume (V) obtained upon measurement of BET specific surface area, from the following equation.

$$D = 4V/S$$

(3) Supporting amount of inorganic particles, water-soluble amine compound, pH control agent and binder resin

A total supporting amount was calculated from a difference between a weight of the fiber structure for deodorization after the fiber structure for deodorization was impregnated with a solution obtained by mixing and dispersing inorganic particles, water-soluble amine compound, a pH control agent and a binder resin, and this was dried, and a weight of a substrate fiber structure before the impregnation and the drying; then it was calculated such that the total supporting amount was multiplied with a charging amount ratio of each component so that it was converted into each supporting amount relative to a whole fiber structure for deodorization.

(4) Equilibrated water content

Regarding an equilibrated water content of inorganic particles, 5 g of a sample which had been pre-dried at a temperature of 50°C for 4 hours with a hot air drier was spread thin on a petri dish, and this was allowed to stand in an experimental chamber adjusted at a temperature of 25°C and a humidity of 75 % for 24 hours and, thereafter, a water content was measured with a water content meter FD-600 manufactured by Kett. A drying temperature was set at 80°C, and a drying time was set at 30 minutes; an equilibrated water content was obtained from a mass before drying and a mass after drying by the following equation.

$$Mr = (W_1 - W_2)/W_2 \times 100$$

wherein Mr: water content
$W_1$: mass before drying (g)
$W_2$: mass after drying (g)

As an equilibrated water content of the fiber structure for deodorization, measurement was performed as in the case of the inorganic particles using three samples which had been longitudinally and transversely cut into 5 cm, and an average was calculated.

(5) pH

The fiber structure for deodorization was immersed in 90 cc of pure water at 20°C to 3 % by weight, this was stirred slightly, and allowed to stand for 10 minutes; a pH of the liquid was measured with a tester pH meter of Lacom. Measurement was performed three times and an average was adopted.

(6) Pressure drop

A sample of a plate-like fiber structure for deodorization was attached to a duct for experiment, and the air at a temperature of 23°C and a humidity of 50 % RH was ventilated to the duct at a velocity of 0.2 m/sec. A pressure difference between an upstream side and a downstream side of a fiber sheet thereupon was measured with a digital manometer MA2-04P manufactured by MODUS.

(7) Ability to remove acetaldehyde

A sample of a plate-like fiber structure for deodorization was attached to a duct for experiment, and the air at a temperature of 23°C and a humidity of 50 % RH was ventilated to the duct at a velocity of 0.2 m/sec. Further, acetaldehyde was added from a further upstream side with a standard gas bombe so that a concentration on an upstream side became 10 ppm. The air was sampled on an upstream side and on a downstream side of a fiber sheet, each acetaldehyde concentration was measured using an infrared light absorbing continuous monitor, and a removal efficiency was calculated by the following equation.

$$\text{Removal efficiency (\%)} = [(C_0 - C)/C_0] \times 100$$

wherein $C_0$: acetaldehyde-concentration on upstream side (ppm)
C: acetaldehyde concentration on downstream side (ppm)

As a removal efficiency, a removal efficiency three minutes after initiation of addition of acetaldehyde was defined as initial removal efficiency, and a removal efficiency post three minutes after was measured with time. In addition, an adsorption amount until a difference between a concentration on an upstream side and a concentration on a downstream side became 5 % was assessed.

(8) Adsorption amount and desorption concentration of odor component other than acetaldehyde

A sample of a plate-like fiber structure for deodorization was attached to a duct, and the air at a temperature of 23°C and a humidity of 50 % RH was ventilated to the duct at a velocity of 0.04 m/sec. Toluene as a representative of a hydrophobic odor component, and acetic acid as a representative of a hydrophilic odor component were warmed to vaporize with a permeater, from a further upstream side. In this respect, both components were adjusted so that an upstream concentration became 80 ppm, respectively. The air was sampled on an upstream side and on a downstream side of the fiber structure for deodorization, concentrations of respective odor components were measured using an infrared light absorbing continuous monitor until a difference between a concentration on an upstream side and a concentration on a downstream side became 5 %, and each gas adsorption amount was assessed.
Then, a sample with an odor component adsorbed thereon was connected to a separately prepared clean line, the sample was taken out, and ventilation of clean air at a temperature of 23°C and a humidity of 50% RH was performed again within one minute at a velocity of 0.04 m/sec. Thereupon, a concentration of an odor component desorbed on a downstream side was measured and a concentration at a time point of maximum was regarded as desorption concentration. This was relatively compared and, thereby, adopted as an index of the effect of suppressing secondary odor emission.

(9) Number average particle diameter of inorganic particles

Regarding a number average particle diameter of inorganic particles, inorganic particles were observed using a transmission electron microscope (TEM H7100 manufactured by Hitachi Corporation), an average of a longest diagonal and a shortest diagonal of individual particles was adopted as particle diameter thereof, and an average of 2000 particles was adopted was a number average particle diameter of inorganic particles.

[Example 1]

(Inorganic particles)

As inorganic particles, hydrophobic porous particles were used. The hydrophobic porous particles were hydrophobic porous silica (Mizupearl K-400 manufactured by MIZUSAWA INDUSTRIAL CHEMICALS, LTD.) having an average particle diameter of 4 $\mu$m, a specific surface area of 250 m$^2$/g, and an average pore diameter of 18 nm, and an equilibrated water content was 5.2 %.

(Water-soluble amine compound)

As water-soluble amine compound, adipic acid dihydrazide (manufactured by Nippon Kasei Chemical Co., Ltd.) was used.

(pH control agent)

In order to adjust an adhesion solution on a weak acidic side, adipic acid was used.

(Substrate fiber structure)

A non-woven fabric of a weight of 50 g/m$^2$ consisting of 16.5% by mass of vinylon having a single fiber fineness of 1.5 dtex, 22 % by mass of vinylon having a single fiber fineness of 7.1 dtex, 16.5 % by mass of polyethylene terephthalate having a single fiber fineness of 2.0 dtex, and 45 % by mass of a phosphorus-based flame retardant-containing acryl resin binder was used as a substrate fiber structure.

(Fiber structure for deodorization)

The substrate fiber structure was impregnated with an aqueous solution in which the hydrophobic porous silica, the adipic acid hydrazide, the pH control agent and an acryl binder resin had been uniformly dispersed at charging amounts described in Table 1 and, thereafter, this was squeezed with a roll, and dried at 100°C for 20 minutes to obtain a fiber structure for deodorization.
The results of assessment of the resulting fiber structure for deodorization are shown in Table 1.

[Example 2]

(Inorganic particles)

The same hydrophobic porous silica as that used in Example 1 was used.

(Water-soluble amine compound)

The same adipic acid dihydrazide as that used in Example 1 was used.

(pH controlling agent)

In order to adjust an adhesion solution on a weak acid side, phosphoric acid melamine was used.

(Substrate fiber construction)

The same structure as that used in Example 1 was used.

(Fiber structure for deodorization)

The substrate fiber structure was impregnated with an aqueous solution in which the hydrophobic porous silica, the adipic acid hydrazide, the pH control agent and the acryl binder resin had been uniformly dispersed at charging amounts described in Table 1 and, thereafter, this was squeezed with a roll, and dried 100°C or 20 minutes to obtain a fiber structure for deodorization.

The results of assessment of the resulting fiber structure for deodorization are shown in Table 1.

[Table 1]

| | | Example 1 | Example 2 |
|---|---|---|---|
| Charging amount into dispersion (mass%) | Hydrophobic porous particle | 10.0 | 10.0 |
| | Adipic acid dihydrazide | 5.0 | 5.0 |
| | pH control agent | 4.0 | 3.0 |
| | Acryl binder resin | 5.0 | 5.0 |
| Carring amount (mass%) | Hydrophobic porous particle | 13.9 | 14.1 |
| | Adipic acid dihydrazide | 6.9 | 7.1 |
| | pH control agent | 5.6 | 4.2 |
| | Acryl binder resin | 6.9 | 7.1 |
| Weight of fiber structure for deodorization (g/m$^2$) | | 75 | 77 |
| Equilibrated water content (mass%) | | 9.3 | 9.1 |
| pH of fiber structure for deodorization | | 4.7 | 5.8 |
| Total pore volume (cc/g) | | 0.029 | 0.031 |
| Ratio of pore volume of 20 nm or less (%) | | 32 | 34 |
| Specific surface area of fiber structure for deodorization (m2/g) | | 7.73 | 7.94 |
| Pressure drop (Pa) | | 8.0 | 8.0 |
| After 3 minutes acetaldehyde removal efficiency (%) | | 41 | 29 |
| Adsorption amount of acetaldehyde (g/m$^2$) | | 1.6 | 1.0 |
| Adsorption amount of toluene (g/m$^2$) | | 0.12 | 0.10 |
| Desorption concentration of toluene (ppm) | | 0.4 | 0.4 |
| Adsorption amount of acetic acid (g/m$^2$) | | 0.92 | 0.96 |
| Desorption concentration of acetic acid (ppm) | | 3.5 | 3.3 |

[Example 3]

(Inorganic particles)

As inorganic particles, poreless ultrafine particles were used. The ultrafine particles were alumina (AEROXIDE Alu-C manufactured by Nippon Aerosil Co. Ltd.) having an average particle diameter of 0.008 $\mu$m, and a specific surface area of 98.21 m$^2$/g, and an equilibrated water content was 4%.

(Water-soluble amine compound)

The same adipic acid dihydrazide as that used in Example 1 was used.

(pH control agent)

In order to adjust an adhesion solution on a weak acidic side, adipic acid was used.

(Substrate fiber structure)

The same structure as that used in Example 1 was used.

(Fiber structure for deodorization)

The substrate fiber structure was impregnated with an aqueous solution in which the alumina, the adipic acid hydrazide, the pH control agent and the acryl binder resin had been uniformly dispersed at charging amounts described in Table 2 and, thereafter, this was squeezed with a roll, and dried at 110°C for 5 minutes to obtain a fiber structure for deodorization.

The results of assessment of the resulting fiber structure for deodorization are shown in Table 2. In addition, it can be determined that, in the resulting fiber structure for deodorization, the inorganic particles together with the water-soluble amine compound formed pores on a fiber surface, from the results of measurement of a BET specific surface area and a pore volume.

[Example 4]

As inorganic particles, poreless ultrafine particles were used. The ultrafine particles were silica (UFP-80 manufactured by DENKI KAGAKU KOGYO KABUSHIKI KAISHA) having an average particle diameter of 0.04 $\mu$m, and a specific surface area of 79.60 m$^2$/g, and an equilibrated water content was 5 %.

(Water-soluble amine compound)

The same adipic acid dihydrazide as that used in Example 1 was used.

(pH control agent)

The same adipic acid as that used in Example 1 was used.

(Substrate fiber structure)

The same structure as that used in Example 1 was used.

(Fiber structure for deodorization)

The substrate fiber structure was impregnated with an aqueous solution in which the silica, the adipic acid hydrazide, the pH control agent and the acryl binder resin had been uniformly dispersed at charging amounts described in Table 2 and, thereafter, this was squeezed with a roll, and dried at 110°C for 5 minutes to obtain a fiber structure for deodorization.

The results of assessment of the resulting fiber structure for deodorization are shown in Table 2. In addition, it can be determined that, in the resulting fiber structure, the inorganic particles together with the water-soluble amine compound formed pores on a fiber surface, from the results of measurements a BET specific surface area and a pore volume.

[Example 5]

As inorganic particles, poreless ultrafine particles were used. The ultrafine particles were silica ((aminosilane-treated) UFP-80 manufactured by DENKI KAGAKU KOGYO KABUSHIKI KAISHA) having an average particle diameter of 0.04 $\mu$m and a specific surface area of 79.80 m$^2$/g, and an equilibrated water content was 4 %.

(Water-soluble amine compound)

The same adipic acid dihydrazide as that used in Example 1 was used.

(pH control agent)

The same adipic acid as that used in Example 1 was used.

(Substrate fiber structure)

The same structure as that used in Example 1 was used.

(Fiber structure for deodorization)

The substrate fiber structure was impregnated with an aqueous solution in which the silica, the adipic acid hydrazide, the pH control agent, and the acryl binder resin had been uniformly dispersed at charging amounts described in Table 2 and, thereafter, this was squeezed with a roll, and dried at 110°C for 5 minutes to obtain a fiber structure for deodorization.

The results of assessment of the resulting fiber structure for deodorization are shown in Table 2. In addition, it can be determined that, in the resulting fiber structure for deodorization, the inorganic particles together with the water-soluble amine compound formed pores on a fiber surface, from the results of measurement of a BET specific surface area and a pore volume.

[Example 6]

As the inorganic particles, the poreless ultrafine particles were used. The ultrafine particles were silica (AEROSIL 200 manufactured by Nippon Aerosil Co. Ltd.) having an average particle diameter of 0.008 $\mu$m, and a specific surface area of 198.2 m$^2$/g, and an equilibrated water content was 5%.

(Water-soluble amine compound)

The same adipic acid dihydrazide as that used in Example 1 was used.

(pH control agent)

The same adipic acid as that used in Example 1 was used.

(Substrate fiber structure)

The same structure as that used in Example 1 was used.

(Fiber structure for deodorization)

The substrate fiber structure was impregnated with an aqueous solution in which the silica, the adipic acid hydrazide, the pH control agent and the acryl binder resin had been uniformly dispersed at charging amounts described in Table 2 and, thereafter, this was squeezed with a roll, and dried at 110°C for 5 minutes to obtain a fiber structure for deodorization.

The results of assessment of the resulting fiber structure for deodorization are shown in Table 2. In addition, it can be determined that, the resulting fiber structure for deodorization, the inorganic particles together with the water-soluble amine compound formed pores on a fiber surface, from the results of measurement of a BET specific surface area and a pore volume.

[Example 7]

As the inorganic particles, poreless ultrafine particles were used. The ultrafine particles were titania (TTO-51(C) manufactured by ISHIHARA SANGYO KAISHA LTD.) having an average particle diameter of 0.02 $\mu$m, and a specific surface area of 55.20 m$^2$/g, and an equilibrated water content was 5%.

(Water-soluble amine compound)

The same adipic acid dihydrazide as that used in Example 1 was used.

(pH control agent)

The same adipic acid as that used in Example 1 was used.

(Substrate fiber structure)

The same structure as that used in Example 1 was used.

(Fiber structure for deodorization)

The substrate fiber structure was impregnated with an aqueous solution in which the titania, the adipic acid hydrazide, the pH control agent and the acryl binder resin had been uniformly dispersed at charging amounts described in Table 2 and, thereafter, this was squeezed with a roll, and dried at 110°C for 5 minutes to obtain a fiber structure for deodorization.

The results of assessment of the resulting fiber structure for deodorization are shown in Table 2. In addition, it can be determined that, in the resulting fiber structure for deodorization, the inorganic particles together with the water-soluble amine compound formed pores on a fiber surface, from the results of measurement of a BET specific surface area and a pore volume.

[Example 8]

(Inorganic particles)

The same alumina as that used in Example 3 was used.

(Water-soluble amine compound)

The same adipic acid dihydrazide as that used in Example 1 was used.

(pH control agent)

The same adipic acid as that used in Example 1 was used.

(Guanidine sulfamate)

As guanidine sulfamete, Apinone -101 manufactured by Sanwa Chemical Co., Ltd. was used.

(Substrate fiber structure)

The same structure as that used in Example 1 was used.

(Fiber structure for deodorization)

The substrate fiber structure was impregnated with an aqueous solution in which the alumina, the adipic acid hydrazide, the pH control agent, the guanidine sulfamate and the acryl binder resin had been uniformly dispersed at charging amounts described in Table 2 and, thereafter, this was squeezed with a roll, and dried at 110°C for 5 minutes to obtain a fiber structure for deodorization.

The results of assessment of the resulting fiber structure for deodorization are shown in Table 2. In addition, it can be determined that, in the resulting fiber structure for deodorization, the inorganic particles together with the water-soluble amine compound formed pores on a fiber surface, from the result of measurement of a BET specific surface area and a pore volume.

[Example 9]

(Inorganic particles)

As inorganic particles, poreless particles were used. The poreless particles were silica (EXCELICA SE-8 manufactured by TOKUYAMA Corp.) having an average particle diameter of 8 $\mu$m, and a pore surface are of 1.52 m$^2$/g, and an equilibrated water content was 5%.

(Water-soluble amine compound)

The same adipic acid dihydrazide as that used in Example 1 was used.

(pH control agent)

The same adipic acid as that used in Example 1 was used.

(Substrate fiber structure)

The same structure as that used in Example 1 was used.

(Fiber structure for deodorization)

The substrate fiber structure was impregnated with an aqueous solution in which the silica, the adipic acid dihydrazide, the pH control agent and the acryl binder resin had been uniformly dispersed at charging amounts described in Table 2 and, thereafter, this was squeezed with a roll and dried at 110°C for 5 minutes to obtain a fiber structure for deodorization

The results of assessment of the resulting fiber structure for deodorization are shown in Table 2.

[Table 2]

| | | Example 3 | Example 4 | Example 5 | Example 6 | Example 7 | Example 8 | Example 9 |
|---|---|---|---|---|---|---|---|---|
| Charging amount into dispersion (mass%) | Inorganic particle | 5.0 | 5.0 | 5.0 | 5.0 | 5.0 | 5.0 | 5.0 |
| | Adipic acid dihydrazide | 2.5 | 2.5 | 2.5 | 2.5 | 2.5 | 2.5 | 2.5 |
| | pH control agent | 1.0 | 1.0 | 1.0 | 1.0 | 1.0 | 1.0 | 1.0 |
| | Guanidine sulfamate | 0.0 | 0.0 | 0.0 | 0.0 | 0.0 | 0.3 | 0.0 |
| | Acryl binder resin | 2.0 | 2.0 | 2.0 | 2.0 | 2.0 | 2.0 | 2.0 |
| Carring amount (mass%) | Inorganic particle | 12 | 13 | 13 | 13 | 13 | 13 | 15 |
| | Adipic acid dihydrazide | 6.1 | 6.4 | 6.4 | 6.4 | 6.6 | 6.4 | 7.3 |
| | pH control agent | 2.4 | 2.5 | 2.5 | 2.5 | 2.6 | 2.6 | 2.9 |
| | Guanidine sulfamate | 0.0 | 0.0 | 0.0 | 0.0 | 0.0 | 0.8 | 0.0 |
| | Acryl binder resin | 4.9 | 5.1 | 5.1 | 5.1 | 5.3 | 5.1 | 5.8 |
| Weight of fiber structure for deodorization (g/m$^2$) | | 70 | 71 | 71 | 71 | 72 | 72 | 75 |
| Equilibrated water content (mass%) | | 6.3 | 9.8 | 8.2 | 9.1 | 4.2 | 4.2 | 7.1 |
| pH of fiber structure for deodorization | | 4.5 | 4.2 | 4.3 | 4.5 | 4.6 | 4.6 | 4.5 |
| Total pore volume (cc/g) | | 0.068 | 0.032 | 0.038 | 0.062 | 0.045 | 0.063 | <0.005 |

(continued)

|  | Example 3 | Example 4 | Example 5 | Example 6 | Example 7 | Example 8 | Example 9 |
|---|---|---|---|---|---|---|---|
| Ratio of pore volume of 20 nm or less (%) | 15 | 19 | 17 | 22 | 16 | 16 | - |
| Specific surface area of fiber structure for deodorization (m2/g) | 6.27 | 5.81 | 6.23 | 9.34 | 3.82 | 6.13 | 0.32 |
| Pressure drop (Pa) | 6.0 | 6.5 | 6.5 | 6.0 | 6.0 | 6.0 | 6.0 |
| After 3 minutes acetaldehyde removal efficiency (%) | 44 | 40 | 46 | 50 | 43 | 55 | 23 |
| Adsorption amount of acetaldehyde (g/m$^2$) | 1.5 | 1.5 | 1.3 | 1.5 | 1.3 | 1.8 | 0.5 |
| Adsorption amount of toluene (g/m$^2$) | 0.08 | 0.09 | 0.13 | 0.08 | 0.09 | 0.08 | 1.02 |
| Desorption concentration of toluene (ppm) | 0.4 | 0.3 | 0.4 | 0.3 | 0.4 | 0.4 | 0.5 |
| Adsorption amount of acetic acid (g/m$^2$) | 2.10 | 1.20 | 1.67 | 2.35 | 1.92 | 2.48 | 1.08 |
| Desorption concentration of acetic acid (ppm) | 2.2 | 3.1 | 2.4 | 3.4 | 2.8 | 2.5 | 0.8 |

[Example 10]

(Deodorizer particles)

As deodorizer particles, deodorizer particles obtained by mixing inorganic particles and an acryl resin binder, and granulating the mixture were used. As the inorganic particles, the same hydrophilic porous silica as that used in Example 1 was used. As the binder, an acryl resin binder having a solid matter of 40% by mass was used. First, a slurry having 20% by mass of hydrophobic porous silica is prepared. The binder is added so that a solid matter of the acryl resin binder becomes 20 parts by mass relative to a solid matter of this hydrophobic porous silica, and the materials are uniformly dispersed to obtain a slurry, which is used as a sample solution. Then, the sample solution was spray-dried using a pressure nozzle-type spray drier to granulate deodorizer particles. The resulting deodorizer particles had a number average particle diameter of 86 μm, a pore surface are of 160 m$^2$/g, and an average pore diameter of 19 nm, and an equilibrated water content was 7.4%.

(Water-soluble amine compound)

The same adipic acid dihydrazide as that used in Example 1 was used.

(pH control agent)

The same adipic acid as that used in Example 1 was used.

(Substrate fiber structure)

The same structure as that used in Example 1 was used.

(Fiber structure for deodorization)

An aqueous solution for adhering a drug, in which the adipic acid hydrazide and the pH control agent had been uniformly dispersed at charging amounts described in Table 3 was prepared, then, the aqueous solution for adhering a drug at the same weight as that of the deodorizer particles was sprayed to the deodorizer particles evenly, and the deodorizer particles were impregnated with the aqueous solution and, further, dried at 100°C for 60 minutes to obtain drug-adhered deodorizer particles . Then, a mixture of the deodorizer particles and a polyolefin thermal adhesive resin powder (Powder Resin 1070 manufactured by TOKYO PRINTING ING MFG Co., Ltd.) at a mass ratio of 4:1 was scattered on a surface of the substrate fiber structure to 100 g/m$^2$ and, further, a spunbond non-woven fabric consisting of a polyethylene terephthalate resin having a weight of 30 g/m$^2$ was further laminated thereon. This was pressed to thermally adhere with a hot plate at 140°C to obtain a fiber structure for deodorization.

The results of assessment of the resulting fiber structure for deodorization are shown in Table 3.

[Table 3]

| | | Example 10 |
|---|---|---|
| Charging amount of adhered drug (mass%) | Adipic acid dihydrazide pH control agent | 6.0 |
| | | 4.0 |
| Composition of drug-adhered deodorizer particle (mass%) | Hydrophobic porous particle | 75.8 |
| | Adipic acid dihydrazide | 5.5 |
| | pH control agent | 3.6 |
| | Acryl binder resin | 15.2 |
| Weight of drug-adhered deodorizer particle (g/m$^2$) | | 80 |
| Weight of thermal adhesive powder (g/m$^2$) | | 20 |
| Weight of fiber structure for deodorization (g/m$^2$) | | 180 |
| Equilibrated water content (mass%) | | 11.4 |
| pH of fiber structure for deodorization | | 5.9 |
| Total pore volume (cc/g) | | 0.150 |
| Ratio of pore volume of 20 nm or less (%) | | 33 |
| Specific surface area of fiber structure for deodorization (m2/g) | | 62.80 |
| Pressure drop (Pa) | | 13.5 |
| After 3 minutes acetaldehyde removal efficiency (%) | | 42 |
| Adsorption amount of acetaldehyde (g/m$^2$) | | 1.7 |
| Adsorption amount of toluene (g/m$^2$) | | 0.32 |
| Desorption concentration of toluene (ppm) | | 0.8 |
| Adsorption amount of acetic acid (g/m$^2$) | | 1.36 |
| Desorption concentration of acetic acid (ppm) | | 3.3 |

[Comparative Example 1]

(Inorganic particles)

As inorganic particle, hydrophilic porous particles were used. The hydrophilic porous particles is hydrophilic porous silica (Cylysia 550 manufactured by FUHI SILYSIA CHEMICAL LTD.) having an average particle diameter of 5μm, a specific surface area of 450 m$^2$/g, and an average pore diameter of 7 nm, and an equilibrated water content was 33%.

(Water-soluble amine compound)

The same adipic acid dihydrazide as that used in Example 1 was used.

(pH control agent)

A pH control agent was not used.

(Substrate fiber structure)

The same structure as that used in Example 1 was used.

(Fiber structure for deodorization)

The substrate fiber structure was impregnated with an aqueous solution in which the hydrophilic porous silica, the adipic acid hydrazide and the acryl binder resin had been uniformly dispersed at charging amounts described in Table 4 and, thereafter, this was squeezed with a roll, and dried at 100°C for 20 minutes to obtain a fiber structure for deodorization.
The results of assessment of the resulting fiber structure for deodorization are shown in Table 4.

[Comparative Example 2]

(Inorganic particles)

As inorganic particles, hydrophilic porous particles were used. The hydrophilic porous particles was porous silica (Cylysia 250 manufactured by FUHI SILYSIA CHEMICAL LTD.) having an average particle diameter of 5.7 $\mu$m, a specific surface area of 288.0 $m^2$/g, and an average pore diameter of 18 nm, and an equilibrated water content was 24%.

(Water-soluble amine compound)

The same adipic acid dihydrazide as that used in Example 1 was used.

(pH control agent)

A pH control agent was not used.

(Substrate fiber structure)

The same structure as that used in Example 1 was used.

(Fiber structure for deodorization)

The substrate fiber structure was impregnated with an aqueous solution in which the porous silica, the adipic acid dihydrazide and the acryl binder resin had been uniformly dispersed at charging amounts described in Table 4 and, thereafter, this was squeezed with a roll, and dried at 110°C for 5 minutes to obtain a fiber structure for deodorization.
The results of assessment of the resulting fiber structure for deodorization are shown in Table 4.

[Table 4]

|  |  | Comparative Example 1 | Comparative Example 2 |
|---|---|---|---|
| Charging amount into dispersion (mass%) | Hydrophilic porous particle | 10.0 | 10.0 |
|  | Adipic acid dihydrazide | 5.0 | 5.0 |
|  | pH control agent | 0.0 | 0.0 |
|  | Acryl binder resin | 5.0 | 4.0 |

(continued)

|  |  | Comparative Example 1 | Comparative Example 2 |
|---|---|---|---|
| Carring amount (mass%) | Hydrophilic porous particle | 16.7 | 15.1 |
|  | Adipic acid dihydrazide | 8.3 | 6.0 |
|  | pH control agent | 0.0 | 0.0 |
|  | Acryl binder resin | 8.3 | 4.6 |
| Weight of fiber structure for deodorization (g/m$^2$) | | 75 | 73 |
| Equilibrated water content (mass%) | | 24.0 | 24 |
| pH of fiber structure for deodorization | | 6.3 | 6.5 |
| Total pore volume (cc/g) | | 0.075 | 0.150 |
| Ratio of pore volume of 20 nm or less (%) | | 95 | 84 |
| Specific surface area of fiber structure for deodorization (m2/g) | | 46.10 | 32.21 |
| Pressure drop (Pa) | | 9.0 | 6.0 |
| After 3 minutes acetaldehyde removal efficiency (%) | | 38 | 51 |
| Adsorption amount of acetaldehyde (g/m$^2$) | | 1.1 | 1.3 |
| Adsorption amount of toluene (g/m$^2$) | | 0.25 | 0.22 |
| Desorption concentration of toluene (ppm) | | 1.1 | 0.9 |
| Adsorption amount of acetic acid (g/m$^2$) | | 4.78 | 4.33 |
| Desorption concentration of acetic acid (ppm) | | 9.4 | 8.9 |

[Comparative Example 3]

(Inorganic particles)

The same hydrophobic porous silica as that used in Example 1 was used.

(Water-soluble amine compound)

The same adipic acid dihydrazide as that used in Example 1 was used.

(pH control agent)

A pH control agent was not used.

(Substrate fiber structure)

The same structure as that used in Example 1 was used.

(Fiber structure for deodorization)

The substrate fiber structure was impregnated with an aqueous solution in which the hydrophobic porous silica, the adipic acid hydrazide and the acryl binder resin had been uniformly dispersed at charging amounts described in Table 5 and, thereafter, this was squeezed with a roll, and dried at 100°C for 20 minutes to obtain a fiber structure for deodorization.
The results of assessment of the resulting fiber structure for deodorization are shown in Table 5.

[Table 5]

| | | Comparative Example 3 |
|---|---|---|
| Charging amount into dispersion (mass%) | Hydrophobic porous particle | 10.0 |
| | Adipic acid dihydrazide | 5.0 |
| | pH control agent | 0.0 |
| | Acryl binder resin | 5.0 |
| Carring amount (mass%) | Hydrophobic porous particle | 16.7 |
| | Adipic acid dihydrazide | 8.3 |
| | pH control agent | 0.0 |
| | Acryl binder resin | 8.3 |
| Weight of fiber structure for deodorization (g/m$^2$) | | 75 |
| Equilibrated water content (mass%) | | 8.9 |
| pH of fiber structure for deodorization | | 7.4 |
| Total pore volume (cc/g) | | 0.034 |
| Ratio of pore volume of 20 nm or less (%) | | 33 |
| Specific surface area of fiber structure for deodorization (m2/g) | | 8.14 |
| Pressure drop (Pa) | | 8.5 |
| After 3 minutes acetaldehyde removal efficiency (%) | | 18 |
| Adsorption amount of acetaldehyde (g/m$^2$) | | 0.35 |
| Adsorption amount of toluene (g/m$^2$) | | 0.12 |
| Desorption concentration of toluene (ppm) | | 0.5 |
| Adsorption amount of acetic acid (g/m$^2$) | | 0.95 |
| Desorption concentration of acetic acid (ppm) | | 3.4 |

[Comparative Example 4]

(Inorganic particles)

Inorganic particles were not used.

(Water-soluble amine compound)

The same adipic acid dihydrazide as that used in Example 1 was used.

(pH control agent)

The same adipic acid as that used in Example 1 was used.

(Substrate fiber structure)

The same structure as that used in Example 1 was used.

(Fiber structure for deodorization)

The substrate fiber structure was impregnated with an aqueous solution in which the adipic acid dihydrazide, the pH control agent, and the acryl binder resin had been uniformly dispersed at charging amounts described in Table 6 and, thereafter, this was squeezed with roll and dried at 110°C for 5 minutes to obtain a fiber structure for deodorization.

The results of assessment of the resulting fiber structure for deodorization are shown in Table 6.

[Table 6]

| | | Comparative Example 4 |
|---|---|---|
| Charging amount into dispersion (mass%) | Inorganic grains | 0.0 |
| | Adipic acid dihydrazide | 5.0 |
| | pH control agent | 4.0 |
| | Acryl binder resin | 4.0 |
| Carring amount (mass%) | Inorganic grains | 0.0 |
| | Adipic acid dihydrazide | 6.2 |
| | pH control agent | 5.0 |
| | Acryl binder resin | 5.0 |
| Weight of fiber structure for deodorization (g/m$^2$) | | 62 |
| Equilibrated water content (mass%) | | 5.4 |
| pH of fiber structure for deodorization | | 4.4 |
| Total pore volume (cc/g) | | <0.005 |
| Ratio of pore volume of 20 nm or less (%) | | - |
| Specific surface area of fiber structure for deodorization (m2/g) | | 0.12 |
| Pressure drop (Pa) | | 5.0 |
| After 3 minutes acetaldehyde removal efficiency (%) | | 23 |
| Adsorption amount of acetaldehyde (g/m$^2$) | | 0.2 |
| Adsorption amount of toluene (g/m$^2$) | | 0.09 |
| Desorption concentration of toluene (ppm) | | 0.4 |
| Adsorption amount of acetic acid (g/m$^2$) | | 0.95 |
| Desorption concentration of acetic acid (ppm) | | 0.7 |

[Comparative Example 5]

The same substrate fiber structure as that used in Example 1 was assessed as it was. Pressure drop of the fiber structure was 5 Pa. In addition, an equilibrated water content of the fiber structure was 5.4% and a pH was 7.4. The assessment results are shown in Table 7.

[Table 7]

| | Comparative Example 5 |
|---|---|
| Weight of fiber structure for deodorization (g/m$^2$) | 50 |
| Equilibrated water content (mass%) | 5.4 |
| pH of fiber structure for deodorization | 7.4 |
| Pressure drop (Pa) | 5.0 |
| After 3 minutes acetaldehyde removal efficiency (%) | 9.0 |
| Adsorption amount of acetaldehyde (g/m$^2$) | 0.08 |
| Adsorption amount of toluene (g/m$^2$) | 0.08 |
| Desorption concentration of toluene (ppm) | 0.4 |
| Adsorption amount of acetic acid (g/m$^2$) | 0.70 |

(continued)

| | Comparative Example 5 |
|---|---|
| Desorption concentration of acetic acid (ppm) | 0.7 |

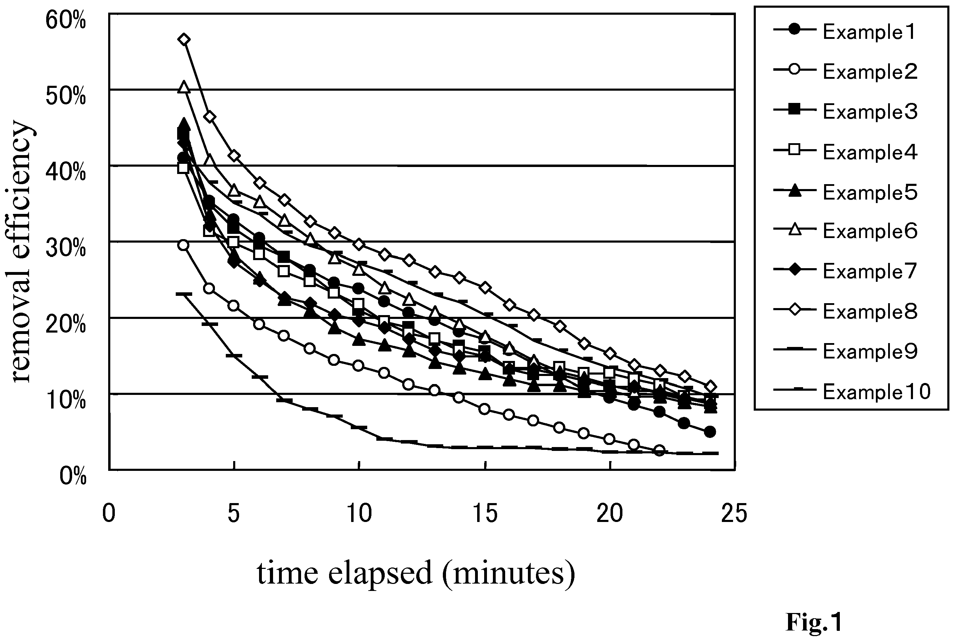

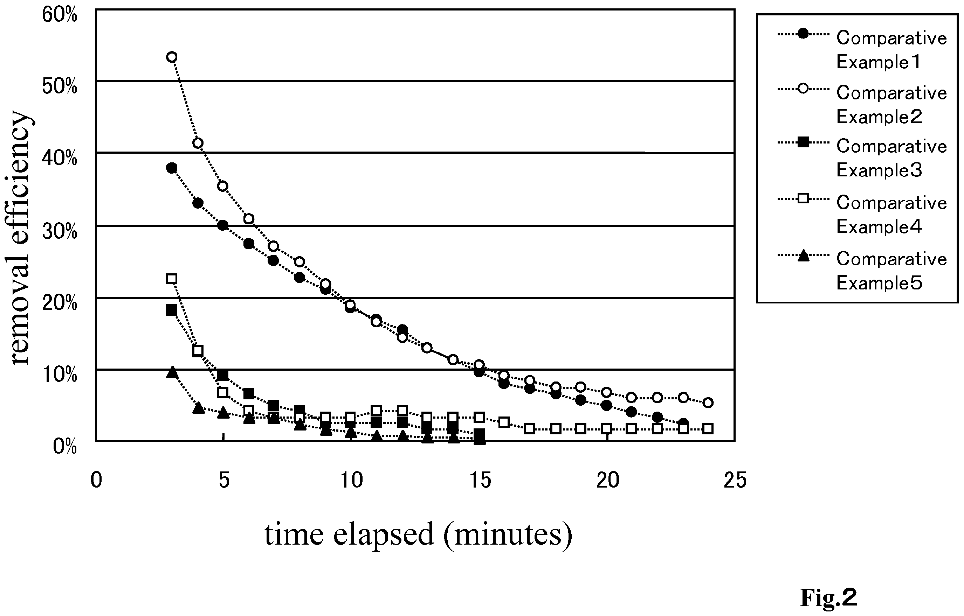

Change in an acetaldehyde removal efficiency of each Example and Comparative Example with time is shown in Fig. 1 and Fig. 2.

In Examples 1 to 10, regarding acetaldehyde, an initial removal efficiency three minutes after initiation of ventilation was as high as 23% to 55%, reduction in a removal efficiency with time was slow and, as a result, an adsorption volume was as great as 0.5 to 1.8 $g/m^2$. Regarding odor components other than acetaldehyde, particularly, in Examples 1 and 2, it is thought that since hydrophobic porous silica whose silanole had been reduced by heat treatment was used, a moisture absorption amount was small, and an adsorption amount of acetic acid was considerably decreased as compared with Comparative Examples 1 and 2 using the previous hydrophilic porous silica, thereby, a desorption concentration was suppressed low. In addition, since hydrophobic porous silica is not extremely hydrophobic to such an extent that toluene is adsorbed, an adsorption amount of toluene was not conversely increased as compared with Comparative Examples 1 and 2 described later; as a result, a desorption concentration was low.

And, in Examples 3 to 9, by adopting poreless particles as inorganic particles, a ratio of a pore volume of 20 nm or less in a total pore volume could be reduced; as a result, both of toluene and acetic acid were small in an adsorption volume, and a desorption concentration could be suppressed low. Particularly in Examples 3 to 8, by using ultrafine particles, a specific surface area became great, and the ability to remove acetaldehyde was particularly excellent.

Additionally, in Example 10 where a processing method was different from Examples 1 to 9, a water-soluble amine-based drug was adhered to deodorizer particles obtained by mixing inorganic particles and the binder resin in advance, and granulating the mixture, and the deodorizer particles were carried in the substrate fiber structure, since the same hydrophobic porous silica as that used in Examples 1 and 2 was adopted, a moisture absorption amount was small, and both of an adsorption amount and a desorption concentration of acetic acid could be suppressed low as in Examples 1 and 2. In addition, also regarding toluene, both of an adsorption amount and a desorption concentration are low.

Like this, it was seen that all of Examples 1 to 10 are materials in which both of a hydrophobic odor component and a hydrophilic odor component are adsorbed with difficulty, while the performance of adsorbing aldehyde is excellent, and secondary odor emission occurs with difficulty.

On the other hand, in Comparative Examples 1 and 2, regarding acetaldehyde, an initial removal efficiency three minutes after initiation of ventilation is as high as 38% and 51%, reduction in a removal efficiency with time is slow; as a result, an adsorption volume is as great as 1.1 $g/m^2$ to 1.3 $g/m^2$. However, in Comparative Examples 1 and 2, due to hygroscopicity of hydrophilic porous silica, an adsorption amount of acetic acid is extremely great; as a result, the situation was brought into the state where a desorption concentration is high, and a desorption odor easily occurs. In addition, in Comparative Examples 3, since hydrophobic porous silica whose silanole had been reduced by heat-treatment was used, a moisture absorption amount was small, and an adsorption amount and a desorption concentration in acetic acid could be suppressed low; however, a pH of the fiber structure for deodorization exceeded 7. For this reason, regarding acetaldehyde, the adsorption performance was low such that an initial removal efficiency three minutes after initiation of ventilation was 18%, and an adsorption volume was 0.35 $g/m^2$. In addition, in Comparative Example 4, since a specific surface area of the fiber structure for deodorization is small, both of toluene and acetic acid were small in an adsorption amount, and a desorption concentration was also low; but since inorganic particles were not used, an absorption volume of acetaldehyde was as low as 0.2 $g/m^2$. In Comparative Example 5 consisted only of the substrate fiber structure, both of toluene and acetic acid were small in an adsorption amount, and a desorption concentration was also low; regarding acetaldehyde, little adsorption performance was possessed such that an initial removal efficiency was 9%, and an adsorption volume was 0.08 $g/m^2$.

Industrial applicability

The fiber structure for deodorization of the present invention can be suitably used in air filter utility. Particularly, in closed spaces such as residencies and vehicle interior, aldehydes contained in tobacco smokes and exhaust gases of automobiles become a problem, but the fiber structure for deodorization of the present invention hardly accumulates odor components except purpose component by physisorption or moisture adsorption. For this reason, even when change in a temperature and a humidity occurs under the air filter use environment, secondary odor emission hardly occurs in narrow rooms. Therefore, such the fiber structure for deodorization can be suitably used in residence and automobile utility.

**Claims**

1.  A fiber structure for deodorization, wherein inorganic particles and water-soluble amine compound are supported in a fiber structure, an equilibrated water content under the environment adjusted at a temperature of 25°C and a relative humidity of 75% is 15% by mass or less, and a pH when the fiber structure is immersed in water to 3% by weight relative to water is 3. 5 to 7, and wherein the inorganic particles are hydrophobic porous particles and the hydrophobic porous particles have an average pore diameter of 2 to 50 nm.

2.  The fiber structure for deodorization according to claim 1, wherein the pH when the fiber structure is immersed in water by 3% by weight relative to water is 3 to 6.5.

3.  the fiber structure for deodorization according to claim 2, wherein the pH when the fiber structure is immersed in water to 3% by weight relative to water is 3 to 5.9.

4.  The fiber structure for deodorization according to any one of claims 1 to 3, wherein an equilibrated water content of the inorganic particles under the environment adjusted at a temperature of 25°C and a relative humidity of 75% is 10% by mass or less.

5.  The fiber structure for deodorization according to any one of claims 1 to 4, wherein the inorganic particles and the water-soluble amine compound form pores on a surface of a fiber, a volume occupied by pores having a pore diameter of 20 nm or less is 40% or less of a total pore volume, and a specific surface area of the fiber structure is 1 to 30 $m^2$/g.

6.  The fiber structure for deodorization according to any one of claims 1 to 5, wherein the inorganic particles have a number average particle diameter of 1 $\mu$m or less and a specific surface area of 15 to 250 $m^2$/g by BET method.

7.  The fiber structure for deodorization according to any one of claims 1 to 6, wherein the inorganic particles contain at least one kind selected from the group consisting of silica, alumina and titania.

8.  The fiber construction for deodorization according to any one of claims 1 to 7, wherein the water-soluble amine compound contains an acid hydrazide compound.

9.  The fiber structure for deodorization according to any one of claims 1 to 8, wherein a water-insoluble pH control agent is contained.

10. The fiber structure for deodorization according to claim 9, wherein the water-insoluble pH control agent is a flame retardant.

11. The fiber structure for deodorization according to any one of claims 1 to 10, wherein the inorganic particles and the water-soluble amine compound are supported on a fiber surface with a binder.

12. The fiber structure for deodorization according to any one of claims 1 to 10, wherein the inorganic particles have a number average particle diameter of 50 to 2000 $\mu$m and is held between fibers in the state where the water-soluble amine compound is adhered to the inorganic particles.

13. An air filter constructed with the fiber structure for deodorization according to any one of claims 1 to 12.

**Patentansprüche**

1.  Faserstruktur zum Desodorieren, wobei anorganische Teilchen und wasserlösliche Aminverbindung in einer Faserstruktur gehalten werden, ein äquilibrierter Wassergehalt in der Umgebung, die auf eine Temperatur von 25 °C und eine relative Feuchtigkeit von 75 % eingestellt ist, 15 Masse-% oder weniger beträgt und ein pH-Wert, wenn die Faserstruktur zu 3 Gew.-% in Bezug auf Wasser in Wasser eingetaucht ist, 3,5 bis 7 beträgt und wobei die anorganischen Teilchen hydrophobe poröse Teilchen sind und die hydrophoben porösen Teilchen einen mittleren Teilchendurchmesser von 2 bis 50 nm aufweisen.

2.  Faserstruktur zum Desodorieren nach Anspruch 1, wobei der pH-Wert, wenn die Faserstruktur zu 3 Gew.-% in Bezug auf Wasser in Wasser eingetaucht ist, 3 bis 6,5 beträgt.

3. Faserstruktur zum Desodorieren nach Anspruch 2, wobei der pH-Wert, wenn die Faserstruktur zu 3 Gew.-% in Bezug auf Wasser in Wasser eingetaucht ist, 3 bis 5,9 beträgt.

4. Faserstruktur zum Desodorieren nach einem der Ansprüche 1 bis 3, wobei ein äquilibrierter Wassergehalt der anorganischen Teilchen in der Umgebung, die auf eine Temperatur von 25 °C und eine relative Feuchtigkeit von 75 % eingestellt ist, 10 Masse-% oder weniger ist.

5. Faserstruktur zum Desodorieren nach einem der Ansprüche 1 bis 4, wobei die anorganischen Teilchen und die wasserlösliche Aminverbindung Poren auf der Oberfläche einer Faser ausbilden, wobei ein von Poren mit einem Porendurchmesser von 20 nm oder weniger belegtes Volumen 40 % oder weniger eines Gesamtporenvolumens ist und wobei eine spezifische Oberfläche der Faserstruktur 1 bis 30 m$^2$/g ist.

6. Faserstruktur zum Desodorieren nach einem der Ansprüche 1 bis 5, wobei die anorganischen Teilchen einen zahlenmittleren Teilchendurchmesser von 1 $\mu$m oder weniger und eine spezifische Oberfläche von 15 bis 250 m$^2$/g durch BET-Verfahren aufweisen.

7. Faserstruktur zum Desodorieren nach einem der Ansprüche 1 bis 6, wobei die anorganischen Teilchen zumindest eine Art ausgewählt aus der Gruppe bestehend aus Siliciumdioxid, Aluminiumoxid und Titandioxid sind.

8. Faserkonstruktion zum Desodorieren nach einem der Ansprüche 1 bis 7, wobei die wasserlösliche Aminverbindung eine Säurehydrazidverbindung enthält.

9. Faserstruktur zum Desodorieren nach einem der Ansprüche 1 bis 8, wobei ein wasserunlösliches pH-Steuerungsmittel enthalten ist.

10. Faserstruktur zum Desodorieren nach Anspruch 9, wobei das wasserunlösliche pH-Steuerungsmittel ein flammenhemmendes Mittel ist.

11. Faserstruktur zum Desodorieren nach einem der Ansprüche 1 bis 10, wobei die anorganischen Teilchen und die wasserlösliche Aminverbindung mit einem Bindemittel auf einer Faseroberfläche gehalten werden.

12. Faserstruktur zum Desodorieren nach einem der Ansprüche 1 bis 10, wobei die anorganischen Teilchen einen zahlenmittleren Teilchendurchmesser von 50 bis 2000 $\mu$m aufweisen und in einem Zustand, in dem die wasserlösliche Aminverbindung an den anorganischen Teilchen anhaftet, zwischen Fasern gehalten werden.

13. Luftfilter, der aus der Faserstruktur zum Desodorieren nach einem der Ansprüche 1 bis 12 gebaut ist.

**Revendications**

1. Structure fibreuse de désodorisation, dans laquelle des particules inorganiques et un composé d'amine hydrosoluble sont supportés dans une structure fibreuse, une teneur en eau équilibrée dans l'environnement ajustée à une température de 25°C et une humidité relative de 75 % est de 15 % en masse ou moins, et un pH lorsque la structure fibreuse est immergée dans de l'eau à 3 % en poids par rapport à l'eau est de 3,5 à 7, et dans laquelle les particules inorganiques sont des particules poreuses hydrophobes et les particules poreuses hydrophobes ont un diamètre de pore moyen de 2 à 50 nm.

2. Structure fibreuse de désodorisation selon la revendication 1, dans laquelle le pH lorsque la structure fibreuse est immergée dans de l'eau à 3 % en poids par rapport à l'eau est de 3 à 6,5.

3. Structure fibreuse de désodorisation selon la revendication 2, dans laquelle le pH lorsque la structure fibreuse est immergée dans de l'eau à 3 % en poids par rapport à l'eau est compris entre 3 et 5,9.

4. Structure fibreuse de désodorisation selon l'une quelconque des revendications 1 à 3, dans laquelle une teneur en eau équilibrée des particules inorganiques sous l'environnement ajusté à une température de 25°C et une humidité relative de 75 % est de 10 % en masse ou moins.

**5.** Structure fibreuse de désodorisation selon l'une quelconque des revendications 1 à 4, dans laquelle les particules inorganiques et le composé d'amine hydrosoluble forment des pores sur une surface d'une fibre, un volume occupé par des pores ayant un diamètre de pore de 20 nm ou moins est de 40 % ou moins d'un volume de pores total, et une surface spécifique de la structure fibreuse est de 1 à 30 $m^2/g$.

**6.** Structure fibreuse de désodorisation selon l'une quelconque des revendications 1 à 5, dans laquelle les particules inorganiques ont un diamètre de particule moyen en nombre de 1 $\mu$m ou moins et une surface spécifique de 15 à 250 $m^2/g$ via un procédé BET.

**7.** Structure fibreuse de désodorisation selon l'une quelconque des revendications 1 à 6, dans laquelle les particules inorganiques contiennent au moins un type choisi dans le groupe constitué par la silice, l'alumine et l'oxyde de titane.

**8.** Construction fibreuse de désodorisation selon l'une quelconque des revendications 1 à 7, dans laquelle le composé d'amine hydrosoluble contient un composé d'hydrazide d'acide.

**9.** Structure fibreuse de désodorisation selon l'une quelconque des revendications 1 à 8, dans laquelle un agent de commande de pH insoluble dans l'eau est contenu.

**10.** Structure fibreuse de désodorisation selon la revendication 9, dans laquelle l'agent de commande de pH insoluble dans l'eau est un ignifugeant.

**11.** Structure fibreuse de désodorisation selon l'une quelconque des revendications 1 à 10, dans laquelle les particules inorganiques et le composé d'amine hydrosoluble sont supportés sur une surface fibreuse avec un liant.

**12.** Structure fibreuse de désodorisation selon l'une quelconque des revendications 1 à 10, dans laquelle les particules inorganiques ont un diamètre de particule moyen en nombre de 50 à 2000 $\mu$m et sont maintenues entre les fibres dans l'état dans lequel le composé d'amine hydrosoluble adhère aux particules inorganiques.

**13.** Filtre à air construit avec la structure fibreuse de désodorisation selon l'une quelconque des revendications 1 à 12.

Fig.1

Fig.2

**REFERENCES CITED IN THE DESCRIPTION**

**Patent documents cited in the description**

- JP 5317703 A
- JP 2007167632 A
- WO 2007074816 A1
- WO 2007066438 A1